# EUROPÄISCHE PATENTSCHRIFT

(11) **EP 3 266 393 B1**
(45) Veröffentlichungstag und Bekanntmachung des Hinweises auf die Patenterteilung: **28.04.2021**
(21) Anmeldenummer: 16178548.0
(22) Anmeldetag: 08.07.2016
(51) Int. Cl.: A61B 17/29, A61B 17/28, A61B 17/30

(54) **CHIRURGISCHES INSTRUMENT**
SURGICAL INSTRUMENT
INSTRUMENT CHIRURGICAL

(43) Veröffentlichungstag der Anmeldung: 10.01.2018
(73) Patentinhaber: Tschida, Peter, 78580 Bärenthal (DE)
(72) Erfinder: Tschida, Peter, 78580 Bärenthal (DE)
(74) Vertreter: Späth, Dieter

(56) Entgegenhaltungen:
- EP-A1- 0 134 251
- EP-A1- 1 897 505
- WO-A1-2017/072224
- DE-A1-102006 007 107
- DE-U1- 20 303 647
- US-A- 5 893 877
- US-A1- 2006 079 933
- US-A1- 2012 277 762
- US-B1- 6 322 578

## Beschreibung

Die Erfindung betrifft ein chirurgisches Instrument mit einer Bedieneinheit zu einer Betätigung des Instruments von Hand mit den Merkmalen des Anspruchs 1. Vorteilhafte Ausgestaltungen sind Gegenstand der Unteransprüche. Das chirurgische Instrument ist zu einer Verwendung in der minimal-invasiven Chirurgie vorgesehen. Das chirurgische Instrument weist beispielsweise eine Schere, eine Zange, einen Greifer oder einen Nadelhalter auf. Die Aufzählung dient der Veranschaulichung und ist nicht abschließend.

Ein derartiges chirurgisches Instrument ist bekannt aus der europäischen Patentanmeldung EP 1 897 505 A1. Das bekannte chirurgische Instrument weist einen Hohlschaft, in dem eine Seele verschiebbar ist, und eine Bedieneinheit zu einer Betätigung des Instruments von Hand auf. Die Bedieneinheit des bekannten chirurgischen Instruments weist zwei Griffe auf, die in einer nicht betätigten Stellung V-förmig schräg in einem spitzen Winkel zueinander stehen und zur Betätigung des chirurgischen Instruments in eine betätigte Stellung zusammendrückbar sind, in der sie parallel zueinander sind. Über zwei Schwenkhebel, deren äußere Enden gelenkig mit einander fernen Enden der Griffe und deren innere Enden gemeinsam gelenkig mit der Seele verbunden sind, wird beim Zusammendrücken der Griffe die Seele im Hohlschaft verschoben. In der nicht betätigten Stellung stehen die beiden Schwenkhebel von der Seele aus gesehen in einem spitzen Winkel von fast 90° V-förmig schräg auseinander. In der betätigten Stellung verlaufen die beiden Schwenkhebel erheblich spitzwinkliger, nämlich fast parallel zueinander. Dadurch ändert sich ein Übersetzungsverhältnis: während eine Geschwindigkeit der Verschiebung der Seele im Hohlschaft beim Zusammendrücken der beiden Griffe abnimmt erhöht sich ihre Kraft.

Die europäische Patentanmeldung EP 0 134 251 A1 offenbart eine medizinische Zange mit einem rohrförmigen Schaft, an dessen einem Ende ein Scherengriff als Bedieneinheit abgewinkelt absteht. Ein Schenkel des Scherengriffs ist fest und ein anderer Schenkel schwenkbar an dem Ende des Schafts angeordnet. Der schwenkbare Schenkel des Scherengriffs ist über einen Hebel schwenkbar mit einer Zahnstange verbunden, die in Längsrichtung des feststehenden Schenkels des Scherengriffs verschiebbar ist und mit einem Schneckenrad mit wendelförmiger Verzahnung kämmt, das koaxial im Ende des Schafts gelagert ist.

Die Patentanmeldung US 2006/079 933 A1 offenbart ein chirurgisches Instrument mit einem koaxial an einem Ende eines rohrförmigen Schafts angeordneten Scherengriff, dessen beide Schenkel V-förmig und symmetrisch schräg nach hinten vom Schaft abstehen. Beide Schenkel des Scherengriffs sind über Hebel gelenkig mit einer Seele verbunden, die sie bei einem Zusammendrücken der Schenkel im Schaft verschieben.

Aufgabe der Erfindung ist eine Verbesserung einer Bedieneinheit eines chirurgisches Instrument vorzuschlagen.

Gelöst wird diese Aufgabe durch ein chirurgisches Instrument mit den Merkmalen des Anspruchs 1. Das erfindungsgemäße chirurgische Instrument ist insbesondere zur Verwendung in der minimal-invasiven Chirurgie vorgesehen. Es weist eine Bedieneinheit zu einer Betätigung des Instruments von Hand auf. Die Bedieneinheit weist ein Grundteil, einen in Bezug auf das Grundteil beweglichen Griff, eine Seele oder ein anderes Antriebsglied, das in Bezug auf das Grundteil beweglich ist, und einen Hebel, der zur Übertragung einer Bewegung des Griffs auf das Antriebsglied mit dem Griff und mit dem Antriebsglied gekoppelt ist, auf. Ein zweites Verbindungsglied verbindet den Griff und den Hebel gelenkig. Das zweite Verbindungsglied ermöglicht beispielsweise eine Schwenklagerung des Griffs und des Hebels am Grundteil der Bedieneinheit bzw. des chirurgischen Instruments ohne mechanische Überbestimmung. Durch die Beweglichkeit in Bezug zum Grundteil sind der Griff, der Hebel und das Antriebsglied auch in Bezug zueinander beweglich. Das Grundteil der Bedieneinheit kann zugleich auch Grundteil oder Teil eines Grundteils des chirurgischen Instruments insgesamt sein.

Eine Anordnung des Griffs in Bezug auf das Grundteil und das Antriebsglied und eine Anordnung des Hebels in Bezug auf den Griff und das Antriebsglied sind so gewählt, dass sich eine Übersetzung der Bedieneinheit bei einer Bewegung des Griffs ändert. Vorzugsweise verkleinert sich ein Weg des Antriebsglieds in Bezug zum Grundteil im Verhältnis zu einer Bewegung des Griffs bei einer Bewegung des Griffs in einer Betätigungsrichtung und umgekehrt erhöht sich eine auf das Antriebsglied ausgeübte Kraft im Verhältnis zu einer auf den Griff ausgeübten Kraft bei der Bewegung des Griffs des chirurgischen Instruments in der Betätigungsrichtung. Mit anderen Worten bewegt sich bei einer Bewegung des Griffs mit konstanter Geschwindigkeit in der Betätigungsrichtung das Antriebsglied am Anfang der Bewegung schneller als am Ende. Mit der Kraft ist es umgekehrt: ein Verhältnis einer Kraft, die die Bedieneinheit auf das Antriebsglied ausübt in Bezug auf eine auf den Griff ausgeübte Kraft erhöht sich während einer Bewegung des Griffs in der Betätigungsrichtung. Die Bedieneinheit des erfindungsgemäßen chirurgischen Instruments ist ein Hebelgetriebe mit sich bei einer Betätigung ändernder Übersetzung.

Erfindungsgemäß ist der Hebel schwenkbar am Grundteil gelagert und der Griff greift mit einem Hebelarm am Hebel und der Hebel greift mit einem Hebelarm am Antriebsglied an, wobei der Hebelarm, mit der der Griff am Hebel angreift länger ist als der Hebelarm, mit der der Hebel am Antriebsglied angreift. Der Hebelarm ist ein Abstand eines Angriffspunkts des Griffs am Hebel bzw. eines Angriffspunkts des Hebels am Antriebsglied von einer Schwenkachse des Hebels am Grundteil. Genau genommen ist der Hebelarm ein Abstand einer Wirkungslinie einer Kraft, mit der der Griff am Hebel und der Hebel am Antriebsglied angreifen, von der Schwenkachse des Hebels am Grundteil. Der längere Hebelarm des Griffs bewirkt eine Kraftübersetzung vom Griff zum Antriebsglied.

Bewegungsarten des Griffs, des Antriebsglieds und des Hebels, nämlich translatorisch und/oder rotatorisch, und Bewegungsrichtungen können verschieden sein und sind vorzugsweise verschieden. Das Antriebsglied ist vorzugsweise in einer Längsrichtung des Grundteils und/oder in seiner Längsrichtung verschiebbar am oder im Grundteil oder einem anderen Element des chirurgischen Instruments geführt. Es überträgt seine Bewegung auf ein Werkzeugteil des chirurgischen Instruments. Das Werkzeugteil ist beispielsweise eine Klinge einer Schere, ein Schenkel einer Pinzette oder eine Backe einer Zange, eines Greifers oder eines Nadelhalters, das das Antriebsglied bei seiner Bewegung verschwenkt. Der Griff ist vorzugsweise schwenkbar mit dem Grundteil verbunden und/oder der Hebel gelenkig mit dem Griff und/oder gelenkig mit dem Werkzeugteil verbunden.

Die Bedieneinheit kann gerade, abgewinkelt oder gekröpft, also mit Versatz zu einer Seite, am chirurgischen Instrument angeordnet sein. Die Bedieneinheit kann vergleichbar einem Pistolengriff mit dem Grundteil als feststehender Handgriff und dem Griff anstelle eines Abzugs ausgebildet sein.

Eine Ausgestaltung der Erfindung sieht einen Winkelhebel als Hebel vor, der schwenkbar am Grundteil gelagert und gelenkig mit dem Griff und gelenkig mit dem Antriebsglied verbunden ist. Dabei liegen eine Schwenkachs einer Schwenklagerung des Winkelhebels am Grundteil und von Achsen gelenkiger Verbindungen des Winkelhebels mit dem Griff und mit dem Antriebsglied nicht auf einer Geraden sondern gehen durch Ecken eines gedachten Dreiecks. Vorzugsweise sind die Schwenkachse und Achsen des Winkelhebels parallel zueinander. Der Griff, der Winkelhebel und das Antriebsglied bilden insbesondere ein ebenes Hebelgetriebe. Bei einer Bewegung des Griffs in der Betätigungsrichtung verschwenkt der Winkelhebel so in Bezug auf den Griff, das Grundteil und das Antriebsglied, dass sich eine Kraft, die der Winkelhebel auf das Antriebsglied ausübt, in Bezug auf eine auf den Griff ausgeübte Kraft in der Betätigungsrichtung erhöht. Umgekehrt ist ein Weg des Antriebsglieds in Bezug auf das Grundteil im Verhältnis zu einem Weg des Griffs am Anfang der Bewegung des Griffs in der Betätigungsrichtung groß und nimmt in der Betätigungsrichtung ab. Durch zwei verschieden lange Hebelarme des Winkelhebels wird eine zusätzliche Kraft- und/oder Wegübersetzung erreicht.

Eine Ausgestaltung der Erfindung sieht ein erstes Verbindungsglied vor, das den Hebel und das Antriebsglied gelenkig verbindet. Das erste Verbindungsglied ermöglicht beispielsweise eine Wandlung einer rotatorischen Bewegung des Hebels in eine translatorische Bewegung des Antriebsglieds.

Zur Erzielung der gewünschten Veränderung der Kraftübersetzung und der entgegengesetzten Veränderung der Wegübersetzung sieht eine Ausgestaltung der Erfindung vor, dass der Hebel bei unbetätigtem chirurgischen Instrument schräg vom Grundteil nach außen in Richtung des Griffs absteht und bei betätigtem chirurgischen Instrument in etwa parallel zum Grundteil verläuft. Ein Winkel zwischen dem Hebel und dem Grundteil wird in der Betätigungsrichtung spitzwinkliger. Anders ausgedrückt wird ein Winkel zwischen einer Bewegungsrichtung des Griffs und dem Hebelarm, mit dem der Griff am Hebel angreift, in der Betätigungsrichtung des chirurgischen Instruments rechtwinkliger. Es verkleinert sich also ein stumpfer oder vergrößert sich ein spitzer Winkel zwischen der Bewegungsrichtung des Griffs und dem Hebelarm, mit dem der Griff am Hebel angreift, bei einer Bewegung in der Betätigungsrichtung.

Vorzugsweise verlängert sich ein wirksamer Hebelarm eines Kraftangriffs des Griffs am Hebel bei einer Bewegung in der Betätigungsrichtung durch eine Änderung einer Winkellage des Hebels.

Eine Ausgestaltung der Erfindung sieht vor, dass das Grundteil ein Rohr ist, in dem das Antriebsglied axial verschieblich aufgenommen ist. Eine andere Ausgestaltung der Erfindung sieht eine Schiebeführung des Antriebsglieds am Grundteil oder einem anderen Element des chirurgischen Instruments beispielsweise mit einer T-Nut vor. Die Aufzählung ist beispielhaft und nicht abschließend.

Eine bevorzugte Ausgestaltung der Erfindung sieht vor, dass der Hebel bei einer Betätigung des chirurgischen Instruments in das Grundteil versenkt oder tiefer versenkt wird. Das Grundteil weist beispielsweise einen Schlitz für den Hebel auf, der vorzugsweise den Hebel seitlich abstützt, um den Hebel bzw. gesamte Hebelgetriebe gegen eine Bewegung quer zur Betätigungsrichtung zu stabilisieren.

Eine Weiterbildung der Erfindung sieht vor, dass das zweite Verbindungsglied, das den Griff und den Hebel gelenkig verbindet, in etwa senkrecht zum Grundteil und/oder zum Griff angeordnet ist, um eine hohe Kraftübertragung vom Griff auf den Hebel zu gewährleisten.

Eine Ausgestaltung der Erfindung sieht zwei bewegliche Griffe und zwei Hebel vor, die entgegengesetzt zueinander und entgegengesetzt in Bezug auf das Grundteil beweglich sind. Insbesondere sind die beiden Griffe und/oder die Hebel symmetrisch bzw. gegensynchron zueinander und zu dem zwischen ihnen angeordneten Grundteil beweglich. Die beiden Griffe können vergleichbar den Schenkeln einer Pinzette oder einer Zange angeordnet sein.

Eine Ausgestaltung der Erfindung sieht eine Rückstellfeder vor, die das chirurgische Instrument entgegen der Betätigungsrichtung, also in einer Öffnungsrichtung oder in Richtung einer nicht betätigten Stellung beaufschlagt. Die Rückstellfeder greift an dem Antriebsglied an. Allgemein ausgedrückt greift die Rückstellfeder nicht über das Hebelgetriebe, sondern an einer Stelle, die sich linear mit dem Antriebsglied bewegt, an. Dadurch unterstützt die sich bei der Betätigung des chirurgischen Instruments vergrößernde Kraftübersetzung des Hebelgetriebes auch das Spannen der Rückstellfeder, eine auf den Griff auszuübende Betätigungskraft zum Spannen der Rückstellfeder ist dadurch kleiner.

Die Erfindung wird nachfolgend anhand zweier in der Zeichnung dargestellter Ausführungsbeispiele näher erläutert. Es zeigen:
- Figur 1: ein chirurgisches Instrument gemäß der Erfindung in perspektivischer Darstellung;
- Figur 2: eine Seitenansicht des chirurgischen Instruments aus Figur 1 ;
- Figur 3: eine Seitenansicht eines Hebelgetriebes des chirurgischen Instruments aus Figur 1; und
- Figur 4: ein zweites Ausführungsbeispiel eines erfindungsgemäßen chirurgischen Instruments in Seitenansicht in nicht betätigter Stellung;
- Figur 5: ein Hebelgetriebe des chirurgischen Instruments aus Figur 4;
- Figur 6: das chirurgische Instrument aus Figur 4 in betätigter Stellung; und
- Figur 7: das Hebelgetriebe des chirurgischen Instruments aus Figur 6.

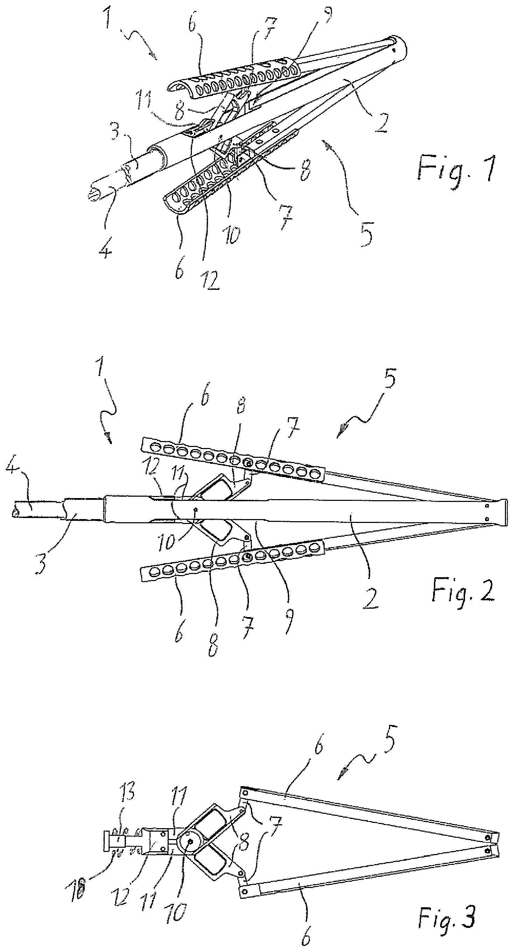

Das in Figuren 1 und 2 dargestellte, chirurgische Instrument 1 ist zur Verwendung in der minimal-invasiven Chirurgie vorgesehen. Es weist ein Rohr als Grundteil 2 und ein weiteres Rohr mit kleinerem Durchmesser als Hohlschaft 3 auf, der gleichachsig und starr mit dem Grundteil 2 verbunden an dessen einem Ende angeordnet ist. In dem Hohlschaft 3 ist eine Stange als Seele 4 verschiebbar aufgenommen.

Zu einer Betätigung von Hand weist das chirurgische Instrument 1 eine Bedieneinheit 5 mit zwei geraden, beweglichen Griffen 6 auf. Enden der Griffe 6 sind schwenkbar an einem Ende des Grundteils 2 gelagert, der sowohl Teil des chirurgischen Instruments 1 als auch der Bedieneinheit 5 ist. In einer gezeichneten, nicht betätigten Stellung des chirurgischen Instruments 1 stehen die beiden Griffe 6 V-förmig in einem spitzen Winkel zueinander schräg nach außen von dem Grundteil 2 ab. Die beiden Griffe 6 sind symmetrisch zum Grundteil 2 und zueinander angeordnet und gegensynchron schwenkbar oder allgemein bewegbar. Im Ausführungsbeispiel sind dem Hohlschaft 3 ferne Enden der Griffe 6 schwenkbar an einem dem Hohlschaft 3 fernen Ende des Grundteils 2 gelagert und stehen schräg in Richtung des Hohlschafts 3 nach außen vom Grundteil 2 ab. Eine umgekehrte Anordnung der beiden Griffe 6 mit ihren Schwenklagern nahe dem Hohlschaft 3 am Grundteil 2 und demzufolge schräg vom Hohlschaft 3 Weg vom Grundteil 2 nach außen abstehend ist ebenso möglich (nicht dargestellt).

Über zweite Verbindungsglieder 7 sind die Griffe 6 gelenkig mit Hebeln verbunden, die als Winkelhebel 8 ausgebildet und spiegelsymmetrisch und in einer Axialebene des Grundteils 2 aneinander anliegend in einem Schlitz 9 des Grundteils 2 angeordnet sind. Die Axialebene des Grundteils 2, in der die beiden Winkelhebel 8 aneinander anliegen, ist parallel zu einer Bewegungsrichtung der Winkelhebel. Der Schlitz 9 stützt die Winkelhebel 8 seitlich ab, so dass sie in Anlage aneinander gehalten sind und sich nicht quer zu einer Schwenkrichtung auseinander bewegen können. In der gezeichneten, nicht betätigten Stellung stehen die beiden Winkelhebel 8 V-förmig in einem spitzen Winkel und nahezu rechtwinklig zueinander schräg nach außen aus dem Schlitz 9 des Grundteils 2 vor. Sie sind um einen Stift 10 schwenkbar gelagert, der radial im Grundteil 2 und quer zum Schlitz 9 angeordnet ist und eine Schwenkachse der beiden Winkelhebel 8 definiert. Die zweiten Verbindungsglieder 7 stehen von den Griffen 6 etwa senkrecht nach innen und greifen gelenkig an dem Stift 10 fernen Enden der Winkelhebel 8 an. Im Ausführungsbeispiel stehen die beiden Winkelhebel 8 entgegengesetzt wie die beiden Griffe 6 schräg vom Grundteil 2 nach außen. Auch hier ist eine umgekehrte Anordnung möglich, so dass die Winkelhebel 8 in gleicher Richtung wie die Griffe 6 schräg nach außen vom Grundteil 2 abstehen (nicht dargestellt).

Zwei erste Verbindungsglieder 11 (Figur 3), die in Längsrichtung des Grundteils 2 im Schlitz 9 angeordnet sind, verbinden die beiden Winkelhebel 8 gelenkig mit einem Querhaupt 12, das längsverschieblich im Schlitz 9 des Grundteils 2 aufgenommen ist und ein mit ihm einstückiges oder starr verbundenes Antriebsglied 13 auf einer den Winkelhebeln 8 abgewandten Seite aufweist. Das Antriebsglied 13 ist lösbar mit der Seele 4 verbunden. Eine Mechanik der Bedieneinheit 5 des erfindungsgemäßen chirurgischen Instruments 1 ist in einer Seitenansicht in Figur 3 ohne das Grundteil 2 dargestellt. Die Mechanik ist ein ebenes Hebelgetriebe, das die schwenkbaren Griffe 6, die Winkelhebel 8, die ersten und die zweiten Verbindungsglieder 11, 7, das Querhaupt 12 und das Antriebsglied 13 umfasst.

Zur Betätigung des chirurgischen Instruments 1 werden die beiden Griffe 6 zusammengedrückt und schwenken über die zweiten Verbindungsglieder 7 die Winkelhebel 8 nach innen in den Schlitz 9 des Grundteils 2 hinein. Durch das Schwenken ziehen die Winkelhebel 8 über die ersten Verbindungsglieder 11 das Querhaupt 12 mit dem Antriebsglied 13 in Richtung des Stifts 10. Die mit dem Antriebsglied 13 verbundene Seele 4 wird dadurch im Hohlschaft 3 bewegt und schließt beispielsweise eine in der Zeichnung nicht sichtbare Schere, Pinzette, Greifer, Zange oder Nadelhalter an einem der Bedieneinheit 1 fernen Ende des Hohlschafts 3 und der Seele 4.

Die Winkelhebel 8 werden als Winkelhebel 8 bezeichnet, weil Angriffspunkte, an denen die ersten und zweiten Verbindungsglieder 11, 7 gelenkig an den Winkelhebeln 8 angreifen mit dem Stift 10, der die Schwenkachse des Winkelhebels 8 definiert, sich an Ecken eines gedachten, näherungsweise rechtwinkligen Dreiecks befinden. Dabei greifen die zweiten Verbindungsglieder 7, die die Winkelhebel 8 mit den Griffen 6 verbinden, mit einem größeren Abstand von dem Stift 10 an den Winkelhebeln 8 an als die ersten Verbindungsglieder 11. Somit greifen die zweiten Verbindungsglieder 7 bzw. die Griffe 6 mit größeren Hebelarmen an den Winkelhebeln 8 an als die ersten Verbindungsglieder 11, wodurch sich eine Kraftübersetzung ergibt. Im Ausführungsbeispiel sind die Hebelarme, mit denen die Griffe 6 an den Winkelhebeln 8 angreifen, ein mehrfaches größer als die Hebelarme, mit denen die Winkelhebel 8 am Querhaupt 12 angreifen. Eine Kraft, die das Hebelgetriebe auf das Antriebsglied 13 und die Seele 4 ausübt, ist dadurch ein Mehrfaches größer als eine Kraft, die auf die Griffe 6 ausgeübt wird.

Außerdem stehen die Winkelhebel 8 in der nicht betätigten Stellung schräg in einem Winkel zu den Griffen 6 nach außen, der beim Schwenken nach innen spitzer wird, bis am Ende der Betätigung die Winkelhebel 8 fast parallel zu den Griffen 6 sind. Genau genommen verläuft der Hebelarm, mit dem die Griffe 6 an den Winkelhebeln 8 angreifen, am Ende der Betätigung des chirurgischen Instruments 1 fast parallel zu den Griffen. Eine Wirkungslinie einer Kraft, die die Griffe 6 auf die Winkelhebel 8 ausüben, verläuft bei unbetätigtem chirurgischen Instrument 1 in einem spitzen oder einem stumpfen Winkel zu den Hebelarmen, mit denen die Griffe 6 an den Winkelhebeln 8 angreifen. Während der Betätigung wird der Winkel zwischen der Wirkungslinie der Kraft und dem Hebelarm stumpfer oder spitzer und ist am Ende der Betätigung rechtwinklig oder fast rechtwinklig. Durch die Winkeländerung ergibt sich eine Kraftübersetzung, die sich während der Betätigung ändert: zu Beginn der Betätigung ist die Kraftübersetzung kleiner und nimmt während der Betätigung zu. Ebenso ändert sich eine Wegübersetzung, bei angenommener konstanter Bewegungsgeschwindigkeit der Griffe 6 nimmt eine Bewegungsgeschwindigkeit des Antriebsglieds 13 ab.

Im Ausführungsbeispiel sind die beiden Winkelhebel 8 mit dem Stift 10 gemeinsam schwenkbar im Grundteil 2 des erfindungsgemäßen chirurgischen Instruments 1 gelagert und üben über die ersten Verbindungsglieder 11 eine Zugkraft auf die Seele 4 aus. Es ist allerdings auch möglich, die Winkelhebel 8 exzentrisch und getrennt voneinander schwenkbar im Grundteil 2 zu lagern, so dass sie eine Druckkraft auf die Seele 4 ausüben (nicht dargestellt).

Nicht zwingend für die Erfindung ist eine in einem Hohlschaft 3 verschiebbare Seele 4, sondern es sind auch zwei gegeneinander verschiebbare Stangen, Schäfte oder dergleichen zur Übertragung der Bewegung des Antriebsglieds 13 gegenüber dem Grundteil 2 zum Schließen und Öffnen einer Schere, Pinzette, Zange, Greifer, Nadelhalter oder dergleichen möglich (nicht dargestellt). Dabei ist eine der beiden Stangen, Schäfte oder dergleichen starr mit dem Antriebsglied 13 und die andere Stange, Schaft oder dergleichen starr mit dem Grundteil 2 verbunden. Vorzugsweise sind die Stangen, Schäfte oder dergleichen verschiebbar miteinander verbunden, beispielsweise mit einer T-Nut und einer eingreifenden T-Leiste.

Zum Öffnen bzw. zum Verbringen in die nicht betätigten Stellung weist das chirurgische Instrument 1 eine Rückstellfeder 18 auf, die in Figur 3 zu sehen ist. Im Ausführungsbeispiel ist die Rückstellfeder 18 eine Schraubendruckfeder, es sind allerdings auch andere Federn oder elastische Elemente als Rückstellfeder 18 möglich. Die Rückstellfeder 18 ist im Grundteil 2 und auf dem Antriebsglied 13 angeordnet, sie stützt sich innen im Grundteil 2 ab und drückt gegen das Querhaupt 12. Bei einer Betätigung des chirurgischen Instruments kommt die sich in der Betätigungsrichtung vergrößernde Kraftübersetzung des Hebelgetriebes auch dem Spannen der Rückstellfeder 18 zugute, durch die die zum Spannen der Rückstellfeder 18 erforderliche, auf die Griffe 6 auszuübende Betätigungskraft weniger stark ansteigt.

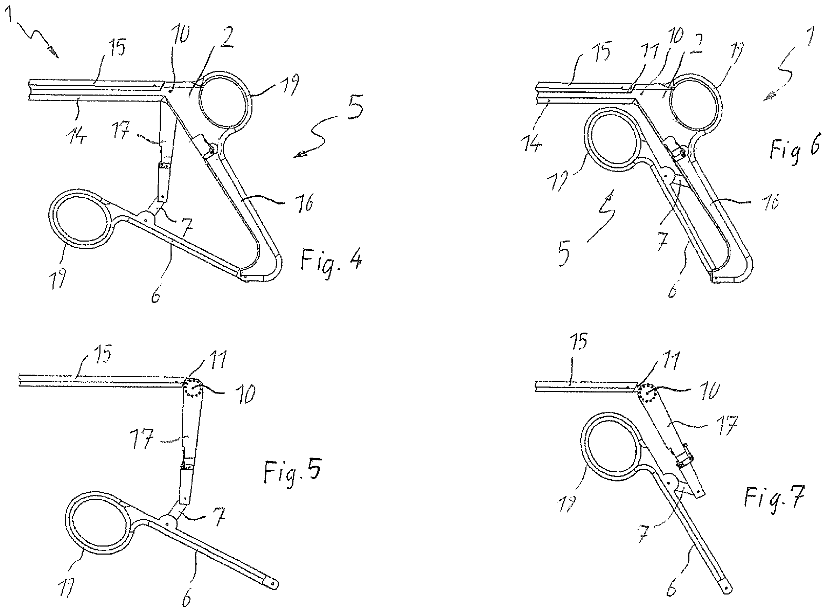

Das in Figuren 4 und 6 gezeichnete, erfindungsgemäße chirurgische Instrument 1 weist einen sogenannten Schiebeschaft anstelle eines Hohlschafts mit Seele auf. Der Schiebeschaft weist einen feststehenden Schaft 14 und eine Schiebestange 15 auf, die am feststehenden Schaft 14 beispielsweise mit einer T-Nut verschiebbar geführt ist.

Bei dem in Figuren 4 und 6 gezeichneten, erfindungsgemäßen chirurgischen Instrument 1 ist eine Bedieneinheit 5 nicht gleichachsig zu dem Schiebeschaft 14, 15 sondern abgewinkelt am Schiebeschaft 14, 15 angeordnet. Von dem feststehenden Schaft 14 steht ein feststehender Griff 16 schräg in einem Winkel ab. Der feststehenden Schaft 14 und der feststehende Griff 16 bilden ein Grundteil 2 des chirurgischen Instruments 1 aus Figuren 4 und 6.

Ein Hebelgetriebe der Bedieneinheit 5 ist asymmetrisch sozusagen wie eine Hälfte des Hebelgetriebes der Bedieneinheit 5 aus Figuren 1 bis 3 ausgebildet. Die Bedieneinheit 5 des in Figuren 4 und 6 dargestellten chirurgischen Instruments 1 weist einen beweglichen Griff 6, ein zweites Verbindungsglied 7, einen Hebel 17 und ein erstes Verbindungsglied 11 auf, die alle auf einer Seite des feststehenden Griffs 16 angeordnet sind. Der Hebel 17 ist kein Winkelhebel, sondern ein (nahezu) gerader, zweiarmiger Hebel. Gerade bedeutet, dass eine Schwenkachse des Hebels 17 und Achsen gelenkiger Verbindungen des zweiten- und des ersten Verbindungsglieds 7, 11 mit dem Hebel 17 auf einer gedachten Geraden liegen. Allerdings ist ein Winkelhebel nicht ausgeschlossen. Zweiarmig bedeutet, dass sich die Angriffspunkte des zweiten- und des ersten Verbindungsglieds 7, 11 am Hebel 17 auf verschiedenen Seiten der Schwenkachse des Hebels 17 befinden, die durch einen Stift 10 definierte Schwenkachse des Hebels 17 befindet sich also zwischen den Angriffspunkten des zweiten- und des ersten Verbindungsglieds am Hebel 17. Ein einarmiger Hebel, bei dem sich die Angriffspunkte des zweiten- und des ersten Verbindungsglieds 7, 11 auf einer gleichen Seite der Schwenkachse des Hebels befinden, die Schwenkachse des Hebels sich also nicht zwischen sondern außerhalb der Angriffspunkte des zweiten- und des ersten Verbindungsglieds 7, 11 am Hebel befindet, ist nicht ausgeschlossen. Der Stift 10, um den der Hebel 17 schwenkbar ist, ist Bereich eines Scheitelpunkts, an dem der feststehende Griff 16 in den feststehenden Schaft 14 übergeht, angeordnet.

In nicht betätigter Stellung, die Figur 4 zeigt, steht das zweite Verbindungsglied 7 in etwa senkrecht zum feststehenden Griff 14 vom beweglichen Griff 6 nach innen und verbindet den beweglichen Griff 6 gelenkig mit einem dem Schiebeschaft 14, 15 fernen Ende des Hebels 17. Ein erstes Verbindungsglied 11 verbindet die Schiebestange 15 gelenkig und mit Abstand von dem Stift 10, der die Schwenkachse des Hebels 17 definiert, mit dem Hebel 17. Das zweite Verbindungsglied 7 ist in Figur 4 verdeckt und deswegen nicht sichtbar, es ist in Figur 5 sichtbar, die ein Hebelgetriebe des chirurgischen Instruments 1 aus Figur 4 in der nicht betätigten Stellung ohne den feststehenden Schaft 14 und ohne den feststehenden Griff 16, die das Grundteil 2 des chirurgischen Instruments 1 bzw. seiner Bedieneinheit 5 bilden, zeigt.

Das Hebelgetriebe der Bedieneinheit 5 des chirurgischen Instruments 1 aus Figur 4 funktioniert in grundsätzlich gleicher Weise wie oben zu Figuren 1 bis 3 erläutert: zu einer Betätigung des chirurgischen Instruments 1 wird der bewegliche Griff 6 zum feststehenden Griff 16 geschwenkt. Er schwenkt dabei über das zweite Verbindungsglied 7 den Hebel 17 zum feststehenden Griff 16, der die Schiebestange 15 entlang des feststehenden Schaft 14 verschiebt. Wird der Stift 10, der den Hebel 17 schwenkbar im Grundteil 2 lagert, oberhalb anstatt unterhalb des Angriffspunkts des ersten Verbindungsglieds 11 angeordnet, übt der Hebel 17 bei der Betätigung des chirurgischen Instruments 1 eine Druckkraft anstatt einer Zugkraft auf die Schiebestange 15 aus. In diesem Fall wäre der Hebel 17 ein einarmiger Hebel.

Ein großes Kraftübersetzungsverhältnis ergibt sich aus einem langen Hebelarm des Hebels 17. Eine Änderung der Kraftübersetzung ergibt sich durch eine Winkelstellung des beweglichen Griffs 6 zum Hebel 17, die sich bei der Betätigung so ändert, dass sich die Kraftübersetzung bei der Betätigung erhöht.

Zur Erläuterung der Figuren 4 bis 7 wird ergänzend Bezug auf die Erläuterungen der Figuren 1 bis 3 genommen. Gleiche Bauteile sind in allen Figuren mit übereinstimmenden Bezugszahlen gekennzeichnet. Die Figuren 6 und 7 zeigen das chirurgische Instrument 1 bzw. sein Hebelgetriebe in betätigter Stellung. Im feststehenden Griff 16 ist wie im Grundteil 2 aus Figuren 1 und 2 ein Schlitz angebracht, der in den Seitenansichten der Figuren 4 und 6 nicht sichtbar ist. In dem Schlitz ist das dem feststehenden Schaft 14 nahe Ende des Hebels 17 aufgenommen, das um den Stift 10 schwenkbar im Grundteil 2 gelagert ist. Bei einer Betätigung des chirurgischen Instruments 1 wird der Hebel 17 in den Schlitz im feststehenden Griff 16 versenkt, wie es in Figur 6 zu sehen ist. Der Schlitz führt den Hebel 17 seitlich und stützt den Hebel 17 seitlich ab.

Das in Figuren 4 und 6 gezeichnete chirurgische Instrument 1 weist keine Rückstellfeder auf sondern wird an Fingerringen 19 am beweglichen Griff 6 und am feststehenden Griff 16 geöffnet.

In Figuren 5 und 7 ist zu sehen, dass der Hebel 17 auf einem Kreis um den Stift 10, um den er schwenkbar ist, angeordnete Löcher aufweist. In dem ein Stift, der das 1. Verbindungsglied 11 gelenkig mit dem Hebel 17 verbindet, durch ein anderes der Löcher gesteckt wird, lässt sich eine Übersetzung des Hebelgetriebes ändern. Die Löcher können auch anders als auf einem Kreis im Hebel 17 angebracht sein. Eine Änderung des Übersetzungsverhältnisses ist in gleicher Weise auch bei dem in Figuren 1 bis 3 gezeichnete chirurgischen Instrument 1 möglich (dort nicht dargestellt).

## Patentansprüche

1. Chirurgisches Instrument zur Verwendung in der minimal-invasiven Chirurgie, mit einer Bedieneinheit (5) zu einer Betätigung des Instruments (1) von Hand, die ein Grundteil (2), einen in Bezug auf das Grundteil (2) beweglichen Griff (6), und einen Hebel (8, 17) aufweist, der gelenkig mit dem Griff (6) verbunden ist und eine Bewegung des Griffs (6) in Bezug auf das Grundteil (2) auf ein Antriebsglied (13) überträgt, das in Bezug auf das Grundteil (2) beweglich und das gelenkig mit dem Hebel (8, 17) verbunden ist, wobei sich eine Übersetzung des Hebels (8, 17) mit einer Bewegung des Griffs (6) in Bezug auf das Grundteil (2) ändert, wobei der Hebel (8, 17) schwenkbar am Grundteil (2) gelagert ist, dass der Griff (6) mit einem längeren Hebelarm am Hebel (8, 17) angreift als das Antriebsglied (13) und **dadurch gekennzeichnet, dass** der Hebel (8, 17) über ein zweites Verbindungsglied (7) gelenkig mit dem Griff (6) verbunden ist.

2. Chirurgisches Instrument nach Anspruch 1, **dadurch gekennzeichnet, dass** der Hebel ein Winkelhebel (8) ist und eine Schwenkachse einer Schwenklagerung des Winkelhebels (8) am Grundteil (2) und Achsen gelenkiger Verbindungen des Griffs (6) mit dem Winkelhebel (8) und des Antriebsglieds (13) mit dem Winkelhebel (8) durch Ecken eines gedachten Dreiecks gehen.

3. Chirurgisches Instrument nach Anspruch 1 oder 2, **gekennzeichnet durch** ein erstes Verbindungsglied (11), das gelenkig mit dem Hebel (8, 17) und gelenkig mit dem Antriebsglied (13) verbunden ist.

4. Chirurgisches Instrument nach einem der vorhergehenden Ansprüche, **dadurch gekennzeichnet, dass** der Hebel (8, 17) bei unbetätigtem chirurgischen Instrument (1) vom Grundteil (2) schräg nach außen in Richtung des Griffs (6) absteht und bei betätigtem chirurgischen Instrument (1) in etwa parallel zum Grundteil (2) verläuft.

5. Chirurgisches Instrument nach einem der vorhergehenden Ansprüche, **dadurch gekennzeichnet, dass** sich ein Hebelarm des Hebels (8, 17) bei einer Betätigung des chirurgischen Instruments (1) durch eine Änderung einer Winkellage des Hebels (8) verlängert.

6. Chirurgisches Instrument nach einem der vorhergehenden Ansprüche, **dadurch gekennzeichnet, dass** das Grundteil (2) ein Rohr ist.

7. Chirurgisches Instrument nach einem der vorhergehenden Ansprüche, **dadurch gekennzeichnet, dass** der Hebel (8, 17) bei einer Betätigung des chirurgischen Instruments (1) in das Grundteil (2) versenkt wird.

8. Chirurgisches Instrument nach einem der vorhergehenden Ansprüche, **dadurch gekennzeichnet, dass** das zweite Verbindungsglied (7) in etwa senkrecht zum Grundteil (2) und/oder zum Griff (6) angeordnet ist.

9. Chirurgisches Instrument nach einem der vorhergehenden Ansprüche, **dadurch gekennzeichnet, dass** der Griff (6) schwenkbar mit dem Grundteil (2) verbunden ist.

10. Chirurgisches Instrument nach einem der vorhergehenden Ansprüche, **dadurch gekennzeichnet, dass** die Bedieneinheit (5) des chirurgischen Instruments (1) zwei besagten Griffe (6) und zwei besagten Hebel (8, 17) und zwei besagten zweiten Verbindungsglieder (7) aufweist, die symmetrisch zu einer Mittelebene angeordnet sind und sich gegensynchron bewegen.

11. Chirurgisches Instrument nach einem der vorhergehenden Ansprüche, **dadurch gekennzeichnet, dass** die Bedieneinheit (5) eine Rückstellfeder (18) aufweist, die auf das Antriebsglied (13) wirkt.

## Claims

1. Surgical instrument for use in minimally invasive surgery, comprising an operating unit (5) intended for operating the instrument (1) by hand and having a main part (2), a handle (6) that is movable relative to the main part (2), and a lever (8, 17) which is articulated to the handle (6) and transmits movement of the handle (6) relative to the main part (2) to a drive member (13) which is movable relative to the main part (2) and articulated to the lever (8, 17), the transmission ratio of the lever (8, 17) changing when the handle (6) moves relative to the main part (2), the lever (8, 17) being pivotably mounted on the main part (2), the handle (6) engaging the lever (8, 17) by a longer lever arm than that which engages the drive member (13), **characterised in that** the lever (8, 17) is articulated to the handle (6) by means of a second connecting member (7).

2. Surgical instrument according to claim 1, **characterised in that** the lever is a bell-crank lever (8) and a pivot axis of a pivot bearing of the bell-crank lever (8) on the main part (2) and axes of articulated connections of the handle (6) to the bell-crank lever (8) and of the drive member (13) to the bell-crank lever (8) pass through corners of an imaginary triangle.

3. Surgical instrument according to either claim 1 or claim 2, **characterised by** a first connecting member (11) which is articulated to the lever (8, 17) and articulated to the drive member (13).

4. Surgical instrument according to any of the preceding claims, **characterised in that** the lever (8, 17) protrudes obliquely from the main part (2) outwards in the direction of the handle (6) when the surgical instrument (1) is not being operated, and extends approximately in parallel with the main part (2) when the surgical instrument (1) is being operated.

5. Surgical instrument according to any of the preceding claims, **characterised in that,** when the surgical instrument (1) is being operated, a lever arm of the lever (8, 17) is extended by an angular position of the lever (8) being changed.

6. Surgical instrument according to any of the preceding claims, **characterised in that** the main part (2) is a tube.

7. Surgical instrument according to any of the preceding claims, **characterised in that** the lever (8, 17) is lowered into the main part (2) when the surgical instrument (1) is being operated.

8. Surgical instrument according to any of the preceding claims, **characterised in that** the second connecting member (7) is arranged approximately perpendicularly to the main part (2) and/or to the handle (6).

9. Surgical instrument according to any of the preceding claims, **characterised in that** the handle (6) is pivotably connected to the main part (2).

10. Surgical instrument according to any of the preceding claims, **characterised in that** the operating unit (5) of the surgical instrument (1) has two of said handles (6), two of said levers (8, 17) and two of said second connecting members (7), which are symmetrical with respect to a central plane and move asynchronously in each case.

11. Surgical instrument according to any of the preceding claims, **characterised in that** the operating unit (5) has a return spring (18) which acts on the drive member (13).

## Revendications

1. Instrument chirurgical conçu pour être utilisé en chirurgie mini-invasive, muni d'une unité de manœuvre (5) dévolue à un actionnement manuel dudit instrument (1) et dotée d'une partie de base (2), d'une poignée (6) mobile par rapport à ladite partie de base (2), et d'un levier (8, 17) qui est relié à ladite poignée (6) de manière articulée et transmet un mouvement, accompli par ladite poignée (6) vis-à-vis de la partie de base (2), à un organe d'entraînement (13) mobile par rapport à ladite partie de base (2) et relié audit levier (8, 17) de manière articulée, sachant qu'une démultiplication dudit levier (8, 17) varie à l'aune d'un mouvement accompli par ladite poignée (6) vis-à-vis de ladite partie de base (2), ledit levier (8, 17) étant monté à pivotement sur ladite partie de base (2), sachant que ladite poignée (6) vient en prise avec ledit levier (8, 17) par un bras de levier plus long que celui dudit organe d'entraînement (13), **caractérisé par le fait que** le levier (8, 17) est relié à la poignée (6), de manière articulée, par l'intermédiaire d'un second organe de liaison (7).

2. Instrument chirurgical selon la revendication 1, **caractérisé par le fait que** le levier est un levier coudé (8), sachant qu'un axe de pivotement d'un support de pivotement dudit levier coudé (8) sur la partie de base (2), et des axes de liaisons articulées de la poignée (6) avec le levier coudé (8), et de l'organe d'entraînement (13) avec ledit levier coudé (8), passent par des coins d'un triangle virtuel.

3. Instrument chirurgical selon la revendication 1 ou 2, **caractérisé par** un premier organe de liaison (11) relié au levier (8, 17) de manière articulée, et à l'organe d'entraînement (13) de manière articulée.

4. Instrument chirurgical selon l'une des revendications précédentes, **caractérisé par le fait que** le levier (8, 17) fait saillie à l'oblique vers l'extérieur au-delà de la partie de base (2) en direction de la poignée (6), à l'état non actionné dudit instrument chirurgical (1), et s'étend à peu près parallèlement à ladite partie de base (2) à l'état actionné dudit instrument chirurgical (1).

5. Instrument chirurgical selon l'une des revendications précédentes, **caractérisé par le fait qu'**un bras du levier (8, 17) s'allonge, lors d'un actionnement dudit instrument chirurgical (1), sous l'effet d'une variation d'une position angulaire dudit levier (8).

6. Instrument chirurgical selon l'une des revendications précédentes, **caractérisé par le fait que** la partie de base (2) est un tube.

7. Instrument chirurgical selon l'une des revendications précédentes, **caractérisé par le fait que** le levier (8, 17) est encaissé dans la partie de base (2) lors d'un actionnement dudit instrument chirurgical (1).

8. Instrument chirurgical selon l'une des revendications précédentes, **caractérisé par le fait que** le second organe de liaison (7) est disposé à peu près perpendiculairement à la partie de base (2) et/ou à la poignée (6).

9. Instrument chirurgical selon l'une des revendications précédentes, **caractérisé par le fait que** la poignée (6) est reliée à la partie de base (2) avec faculté de pivotement.

10. Instrument chirurgical selon l'une des revendications précédentes, **caractérisé par le fait que** l'unité de manœuvre (5) dudit instrument chirurgical (1) compte deux poignées (6) du type précité, deux leviers (8, 17) du type précité et deux seconds organes de liaison (7) du type précité, qui sont agencés symétriquement par rapport à un plan médian et se meuvent en opposition synchrone.

11. Instrument chirurgical selon l'une des revendications précédentes, **caractérisé par le fait que** l'unité de manœuvre (5) est pourvue d'un ressort de rappel (18) agissant sur l'organe d'entraînement (13).
